# EUROPÄISCHE PATENTSCHRIFT

(11) **EP 4 419 050 B1**
(45) Veröffentlichungstag und Bekanntmachung des Hinweises auf die Patenterteilung: **30.07.2025**
(21) Anmeldenummer: 22708343.3
(22) Anmeldetag: 25.01.2022
(51) Int. Cl.: A61F 5/44, A61F 5/445

(54) **STOMABEUTEL-ZUBEHÖR**
STOMA BAG ACCESORY
ACCESSOIRE POUR POCHES DE STOMIE

(43) Veröffentlichungstag der Anmeldung: 28.08.2024
(73) Patentinhaber: Lohmann GmbH & Co. KG, 56567 Rheinland-Pfalz Neuwied (DE); Covestro Deutschland AG, 51373 Leverkusen (DE); Witte Technology GmbH, 48163 Münster (DE)
(72) Erfinder: LUTZ, Volker, 56564 Neuwied (DE); BÜSCHEL, Gerd, 18211 Admannshagen-Bargeshagen (DE); KOSSEL, Klas-Moritz, 27283 Verden (DE); RENNERS, Philip, 48147 Münster (DE)
(86) Internationale Anmeldenummer: PCT/EP2022/051609
(87) Internationale Veröffentlichungsnummer: WO 2023/143697

(56) Entgegenhaltungen:
- WO-A1-2019/120448
- WO-A1-2022/015649
- US-A1- 2017 007 440
- US-A1- 2017 140 103
- US-A1- 2018 233 235
- US-A1- 2021 244 563

## Beschreibung

Die Erfindung betrifft ein Stomabeutel-Zubehör nach dem Anspruch 1 sowie ein Verfahren zur Anwendung eines Stomabeutel-Zubehörs nach dem Anspruch 12.

Aus dem Stand der Technik ist bekannt, Stomabeutelvorrichtungen mit integrierten Sensoren auszustatten, welche dazu vorgesehen sind, Leckagen der Stomabeutelvorrichtung zu detektieren. Die Integration der Sensoren verhindert allerdings ein einfaches Nachrüsten von Stomabeutelvorrichtungen, bei welchen die Sensoren nicht bereits im Herstellungsprozess integriert wurden. Ferner besteht keine Möglichkeit, die Sensoren von der Stomabeutelvorrichtung zu entfernen und erneut anzubringen, wodurch beispielsweise bei einer Beschädigung der Sensoren kein einfaches Austauschen selbiger möglich ist.

Aus der WO 2022/015649 A1 ist ein Beispiel für solche Stomabeutelvorrichtungen mit integrierten Leckage-Sensoren bekannt. Die Sensoren sind spiralförmig um das Stoma angeordnet und zwischen einem Substrat und einer Hydrokolloidplatte fixiert.

Die Aufgabe der Erfindung besteht bevorzugt darin, die Sensoren auf eine Weise bereitzustellen, welche in der Konstruktion und/oder Anwendung eine erhöhte Flexibilität und Anpassbarkeit ermöglicht. Die Aufgabe wird erfindungsgemäß durch die Merkmale der Ansprüche 1 und 12 gelöst, während vorteilhafte Ausgestaltungen und Weiterbildungen der Erfindung den Unteransprüchen entnommen werden können. Es wird ein Stomabeutel-Zubehör zur Verwendung mit zumindest einer Stomabeutelvorrichtung vorgeschlagen, mit zumindest einem Sensorelement zur Erkennung einer Leckage der Stomabeutelvorrichtung in zumindest einem Anwendungszustand und mit zumindest einem Klebeelement zur lösbaren Verbindung mit der Stomabeutelvorrichtung in dem Anwendungszustand.

Durch eine derartige Ausgestaltung kann eine Konstruktion bereitgestellt werden, welche einen erhöhten Komfort und eine verbesserte Flexibilität bietet. Vorteilhaft ist, dass es für den Anwender wählbar ist, wann und wie oft er das Stomabeutel-Zubehör an die Stomabeutelvorrichtung anbringt. Besonders vorteilhaft können bereits am Benutzer angebrachte Stomabeutelvorrichtungen nachträglich und ohne die Notwendigkeit von speziellen Werkzeugen oder ausgebildetem Personal mit dem Stomabeutel-Zubehör ausgestattet werden. Beispielsweise könnte das Stomabeutel-Zubehör vor dem Ausüben einer sportlichen Tätigkeit mit der Stomabeutelvorrichtung verbunden und im Anschluss an die Tätigkeit wieder entfernt werden. Ferner können die Stomabeutelvorrichtung und das Stomabeutel-Zubehör separat angeboten werden, wodurch für Benutzer, die nur selten Bedarf für das Stomabeutel-Zubehör haben, ein unnötiger dauerhafter Aufpreis vermieden werden kann.

Vorzugsweise ist das Stomabeutel-Zubehör beliebig oft mit der Stomabeutelvorrichtung verbindbar und von dieser entfernbar. Vorteilhaft ist das Stomabeutel-Zubehör mit verschiedenen, zueinander identischen, Stomabeutelvorrichtungen, besonders vorteilhaft mit verschiedenen, zueinander unterschiedlichen, Stomabeutelvorrichtungen, verbindbar. Bevorzugt ist das Stomabeutel-Zubehör an Stomabeutelvorrichtungen, welche zueinander unterschiedliche Größen aufweisen, anpassbar. Besonders bevorzugt ist das Stomabeutel-Zubehör zur Verwendung mit unterschiedlichen Stomabeutelvorrichtungen dehnbar und/oder faltbar. Ferner bevorzugt kann das Stomabeutel-Zubehör in verschiedenen Größen und/oder Formen ausgebildet sein, welche jeweils zur Verwendung mit einer bestimmten Art von Stomabeutelvorrichtung vorgesehen sind.

Die Stomabeutelvorrichtung umfasst bevorzugt zumindest eine Öffnung umgeben von einer Hydrokolloidplatte, welche in dem Anwendungszustand auf die Haut eines Benutzers aufgeklebt ist und ein Stoma des Benutzers umgibt, sowie einen Stomabeutel, welcher mit der Hydrokolloidplatte verbunden und dazu vorgesehen ist, aus dem Stoma austretende Stoffe aufzufangen. Die Stomabeutelvorrichtung ist bevorzugt zu einer langanhaltenden Anwendung vorgesehen, kann jedoch, beispielsweise im Fall einer Beschädigung, entfernt und erneut angebracht oder durch eine neue Stomabeutelvorrichtung ersetzt werden. Bei einer Leckage, welche beispielsweise durch eine Stomablockade verursacht werden kann, treten üblicherweise zwischen der Hydrokolloidplatte und der Haut des Benutzers Stoffe aus, welche zu Hautirritationen, Entzündungen und Blamagen des Benutzers führen können und Geruchsbelästigungen mit sich bringen. Die Öffnung ist in den meisten kommerziell erhältlichen Stomabeutelvorrichtungen kreisförmig ausgestaltet.

Das Sensorelement weist bevorzugt zumindest eine Leiterbahn und besonders bevorzugt zumindest zwei Leiterbahnen zur Detektion der Leckage auf. Es wäre möglich, dass die mindestens eine Leiterbahn aus Kupfer und/oder Aluminium besteht. Vorzugsweise ermittelt das Sensorelement die Leckage durch eine konstante Messung einer lokalen Feuchtigkeit. Das Sensorelement könnte die lokale Feuchtigkeit mittels einer kapazitiven Messung und/oder einer Messung der elektrischen Leitfähigkeit in einem Nahbereich der Leiterbahn ermitteln. Bevorzugt misst das Sensorelement die lokale Feuchtigkeit in einer Sensorfläche, welche als eine zwischen zwei Leiterbahnen angeordnete Fläche des Substrats ausgebildet ist. Die Form der mindestens einen Leiterbahn des Sensorelements kann beliebig ausgebildet sein, beispielsweise könnte die Leiterbahn offen oder geschlossen sein. Ferner könnte die Leiterbahn umkehrfrei sein oder zumindest einen Umkehrpunkt aufweisen, an welchem sich zwei parallel zueinander verlaufende Abschnitte der Leiterbahn treffen. Außerdem könnte die Leiterbahn zu einer Verbesserung der Erkennung der Leckage zumindest einen Vorsprung und/oder zumindest einen mäanderartig verlaufenden Abschnitt aufweisen. Vorteilhaft sind benachbarte Sensoren zumindest abschnittsweise einander umgreifend ausgebildet.

Der Anwendungszustand soll als ein Zustand verstanden werden, in welchem die Stomabeutelvorrichtung auf der Haut des Benutzers aufgeklebt und das Stomabeutel-Zubehör mit der Stomabeutelvorrichtung verbunden ist. Bevorzugt ist der Anwendungszustand ein Zustand, in dem sowohl die Stomabeutelvorrichtung als auch das Stomabeutel-Zubehör sämtliche ihrer Funktionen bereitstellen.

Bevorzugt weist das Klebeelement zumindest einen Klebebereich auf, der bevorzugt einen Klebstoff enthält, ganz bevorzugt einen Klebstoff aus der Gruppe der Thermoplaste oder Elastomere. Bevorzugt ist der Klebebereich so ausgestaltet, dass er flexibel mit verschiedenen Arten von Stomabeutelvorrichtungen kompatibel nutzbar ist. Bevorzugt sind abhängig von der Größe und/oder Form der Stomabeutelvorrichtung verschiedene Teilflächen des Klebebereichs zur Verbindung mit der Stomabeutelvorrichtung. benutzbar. Bevorzugt bildet der Klebebereich das gesamte Klebeelement aus. Alternativ weist das Klebeelement zumindest einen weiteren, nichtklebenden Bereich auf. Das Klebeelement weist bevorzugt eine Breite in einem Bereich von 1 bis 50 cm, weiter bevorzugt im Bereich von 2 bis 20 cm, besonders bevorzugt im Bereich von 5 bis 10 cm auf. Das Klebeelement weist bevorzugt eine Länge in einem Bereich von 2 bis 100 cm, weiter bevorzugt im Bereich von 5 bis 70 cm, besonders bevorzugt im Bereich von 10 bis 50 cm auf.

Bevorzugt weist das Klebeelement eine Atmungsaktivität von mindestens 300 g/m²/24h auf. Hierdurch können eine Hautatmung ermöglicht und auch bei längerer Anwendung des Stomabeutel-Zubehörs Hautschäden vermieden werden. Der Klebebereich weist bevorzugt einen Klebstoff ausgewählt aus der Gruppe bestehend aus einem Silikon-Klebstoff, einem Acrylat-Klebstoff, einem Polyurethan-Klebstoff, einem Kautschuk-Klebstoff sowie Mischungen aus mindestens zwei hiervon und/oder weitere, aus dem Bereich der medizinischen Pflaster und Wundauflagen bekannte Klebstoffe auf oder besteht daraus.

Unter der Verbindung des Stomabeutel-Zubehörs mit der Stomabeutelvorrichtung soll ein Kleben des Klebeelements an die Hydrokolloidplatte verstanden werden. Bevorzugt fixiert die Verbindung mittels dem Klebeelement das Stomabeutel-Zubehör und die Stomabeutelvorrichtung relativ zueinander.

Das Stomabeutel-Zubehör weist zumindest ein Substrat, welches das Sensorelement trägt, und zumindest einen Absorber auf, wobei das Sensorelement bei einer Betrachtung senkrecht auf eine Haupterstreckungsebene des Substrats zwischen dem Substrat und dem Absorber angeordnet ist. Es wäre denkbar, dass das Substrat als eine Leiterplatte ausgebildet ist. Darunter, dass das Substrat das Sensorelement trägt, soll verstanden werden, dass das Sensorelement unlösbar mit dem Substrat verbunden ist. Beispielsweise könnte das Sensorelement mit dem Substrat verschweißt, gelötet, auf das Substrat aufgeklebt und/oder an dem Substrat festgenietet sein. Der Absorber ist bevorzugt dazu vorgesehen, bei einer Leckage austretende Flüssigkeiten zu absorbieren. Vorteilhaft detektiert das Sensorelement die von dem Absorber absorbierte Flüssigkeit, um zu ermitteln, ob eine Leckage vorliegt. Das Sensorelement könnte bei der senkrechten Betrachtung vollständig zwischen dem Substrat und dem Absorber angeordnet sein. Bevorzugt weist das Sensorelement zumindest einen Schnittstellenbereich auf, welcher zwischen dem Substrat und dem Absorber herausragt und besonders bevorzugt mit einem Lesegerät, bevorzugt lösbar, verbindbar ist. Das Lesegerät ist bevorzugt zu einer Auswertung der Messdaten des Sensorelements vorgesehen. Vorteilhaft ist in dem Lesegerät ein Feuchtigkeits-Schwellwert gespeichert, bei dessen Überschreitung das Lesegerät besonders vorteilhaft einen Alarm an den Benutzer auslöst. Der Alarm könnte beispielsweise als ein optischer Alarm, ein akustischer Alarm und/oder ein Vibrationsalarm ausgebildet sein. Es wäre möglich, dass das Lesegerät den Alarm selbst ausgibt. Alternativ oder zusätzlich könnte das Lesegerät eine Kommunikationseinheit aufweisen, welche dazu vorgesehen ist, bei einem Überschreiten des Feuchtigkeits-Schwellwerts ein Signal an ein externes Gerät, wie beispielsweise ein Smartphone des Benutzers oder eine telemedizinische Vorrichtung, zu senden, woraufhin das externe Gerät den Alarm ausgibt. Die Kommunikationseinheit weist vorzugsweise zumindest einen kabellosen Sender auf, welcher beispielsweise als ein Funksender, Bluetooth-Sender und/oder Infrarotsender ausgebildet sein könnte. Unter einer "Haupterstreckungsebene" eines Körpers soll eine Ebene verstanden werden, welche von allen möglichen Ebenen den größten Teil des Körpers schneidet. Vorzugsweise sind das Substrat und der Absorber in einem Ruhezustand, in welchem das Substrat und der Absorber auf einer flachen Oberfläche liegen und weder geknickt noch gefaltet sind, schichtförmig ausgebildet. Unter einem "schichtförmigen" Körper soll ein Körper verstanden werden, dessen Länge und Breite mindestens das Zehnfache, vorteilhaft mindestens das Fünfzehnfache und besonders vorteilhaft mindestens das Zwanzigfache seiner Dicke entspricht. Bevorzugt verläuft die Haupterstreckungsebene des Substrats parallel zu einer Länge und Breite des Substrats in dem Ruhezustand und ist bevorzugt parallel zu einer Haupterstreckungsebene des Absorbers ausgerichtet. Hierdurch kann gewährleistet werden, dass eine bei einer Leckage austretende Flüssigkeit in den Nahbereich des Sensorelements gelangt und dort detektiert wird. Vorteilhaft kann der Absorber gleichzeitig dazu dienen, die austretende Flüssigkeit zurückzuhalten und/oder zu filtern, bis die Leckage behoben wurde oder sich der Benutzer aus dem öffentlichen Raum zurückziehen konnte.

In einer bevorzugten Ausführungsform des Stomabeutel-Zubehörs ist das Sensorelement als ein aufgedruckter Sensor ausgebildet und das Substrat weist zumindest eine, bevorzugt zwei, besonders bevorzugt alle der folgenden Eigenschaften auf:
1) eine Bruchdehnung ≥ 300 % und ≤ 800 %, bevorzugt ≥ 350 % und ≤ 750 %, besonders bevorzugt ≥ 400 % und ≤ 700 %,
2) eine Bruchspannung ≥ 30 MPa und ≤ 100 MPa, bevorzugt ≥ 35 MPa und ≤ 95 MPa, besonders bevorzugt ≥ 40 MPa und ≤ 90 MPa;
3) Beinhalten eines Polymers ausgewählt aus der Gruppe bestehend aus einem Polyester, einem Polycarbonat, einem Co-Polycarbonat, einem Polyurethan, einem thermoplastischen Polyurethan, einem Acrylnitril-Butadien-Styrol-Copolymer, einem Polyethylenterephthalat, einem Polyamin, einem Polyamid, einem Polystyrol und einem Polysiloxan oder Blends oder Mischungen aus mindestens zwei hiervon.

Bevorzugt besteht das Sensorelement aus einer anorganischen oder organischen Tinte, welche bevorzugt vor einem Druckprozess als Lösung, Dispersion oder Suspension von leitfähigen Mikropartikeln und/oder Nanopartikeln ausgebildet ist. Die Mikropartikel und/oder Nanopartikel können zum Beispiel Polyanilin, Silber, Gold und/oder Kohlenstoff oder Mischungen aus mindestens zwei hiervon aufweisen. Beispielsweise kann das Sensorelement mittels eines 2D-Druckverfahrens, wie dem Siebdruck oder Tintenstrahldruck auf das Substrat gedruckt sein. Denkbar wäre auch, dass das Sensorelement mittels eines 3D-Druckverfahrens, wie der Stereolithografie, dem 3D-Siebdruck oder dem Polyjet Modeling auf das Substrat gedruckt sein und bevorzugt eine vordefinierte dreidimensionale Form aufweisen könnte. Die genannten Werte der Bruchdehnung und Bruchspannung richten sich nach der Norm DIN EN ISO 527-1:2016-06. Es wäre denkbar, dass das Substrat vollständig aus einem der genannten Polymere besteht, alternativ könnte das Substrat aus einer Mischung von zumindest zwei der Polymere bestehen. Bevorzugt beinhaltet das Substrat eine Mischung von thermoplastischem Polyurethan und einem Acrylnitril-Butadien-Styrol-Copolymer oder besteht daraus, wobei ein Gewichtsanteil des Acrylnitril-Butadien-Styrol-Copolymers vorzugsweise 5-40 Gew.-%, bezogen auf das Gesamtgewicht des Substrats, beträgt. Alternativ beinhaltet das Substrat eine Mischung aus einem thermoplastischen Polyurethan und einem Polystyrol oder besteht daraus, wobei ein Gewichtsanteil des Polystyrols vorzugsweise 5-40 Gew.-%, bezogen auf das Gesamtgewicht des Substrats, beträgt. Vorteilhaft ist das Substrat zumindest in einem Teilbereich, welcher frei von dem Sensorelement ist, in seiner Erstreckung anpassbar. Bevorzugt ist die Erstreckung des Substrats durch zumindest einen Schneidprozess und/oder zumindest einen Stanzprozess anpassbar. Hierdurch kann eine flexible und auf einfache Weise eine skalierbare Konstruktion des Stomabeutel-Zubehörs erreicht werden. Vorteilhaft kann eine Anpassung des Stomabeutel-Zubehörs, beispielsweise an ein neues Design der Stomabeutelvorrichtung, mit der das Stomabeutel-Zubehör verwendet werden soll, auf einfache und kostengünstige Weise bereitgestellt werden. Besonders vorteilhaft kann sich das Stomabeutel-Zubehör durch die flexible Ausgestaltung des Substrats an eine Form des Hautbereichs des Benutzers, in dem es zur Anwendung kommt, anpassen, um unabhängig von dem Anwendungsort und dem Benutzer eine sichere Detektion von Leckagen zu gewährleisten.

In einer bevorzugten Ausführungsform des Stomabeutel-Zubehörs weist der Absorber zumindest eine, bevorzugt beide der folgenden Eigenschaften auf:
1) eine maximal aufnehmbare Flüssigkeitsmenge von ≥ 40 ml, bevorzugt ≥ 50 ml,
2) eine Porengröße von ≤ 120 µm, bevorzugt ≤ höchstens 100 µm.

Bevorzugt ist der Absorber als ein Superabsorber ausgebildet und weist beispielsweise ein Hydrogel auf. Besonders bevorzugt weist der Absorber zumindest einen Vliesstoff und/oder Schaum auf. Weiterhin bevorzugt weist der Absorber zumindest einen Zellstoff auf. Bevorzugt kann der Absorber zumindest ein zusätzliches Funktionsmaterial aufweisen. Bevorzugt weist das Funktionsmaterial eine Geruchsdämmung bereitstellen und Aktivkohle und/oder Titandioxid auf. Hierdurch kann eine Detektion der Leckage und ein Verhindern des Entweichens von Flüssigkeiten und/oder Gerüchen auch bei größeren Leckagen gewährleistet werden.

Bevorzugt steht der Absorber bei der senkrechten Betrachtung über das Substrat hervor. Bevorzugt weist der Absorber einen zusätzlichen Teilbereich auf, welcher bezüglich der Stomabeutelvorrichtung hinter dem Sensorelement angeordnet ist, um auch nach der Detektion der Leckage zusätzliche Flüssigkeit aufnehmen zu können. Bevorzugt ist der zusätzliche Teilbereich zu einer Verbindung mit dem Klebeelement vorgesehen. Alternativ oder zusätzlich kann das Substrat Ausnehmungen aufweisen, welche bei der senkrechten Betrachtung benachbart zu der mindestens einen Leiterbahn des Sensorelements angeordnet und zu einer Verbindung mit dem Klebeelement vorgesehen sind. Hierdurch kann auf einfache Weise eine Fixierung des Absorbers und des Substrats relativ zueinander erreicht werden.

Besonders bevorzugt sind das Substrat und der Absorber bei der senkrechten Betrachtung zumindest im Wesentlichen zueinander deckungsgleich ausgebildet. Darunter, dass der Absorber und das Substrat zumindest im Wesentlichen zueinander deckungsgleich sind, soll verstanden werden, dass sich die Flächen des Absorbers und des Substrats in der senkrechten Betrachtung mindestens zu 90 %, bevorzugt mindestens zu 95 % und besonders bevorzugt vollständig überlappen, bezogen auf eine Gesamtfläche des Substrats. Bevorzugt sind der Absorber, das Substrat und das Sensorelement relativ zueinander in einer Sandwichbauweise angeordnet, wobei besonders bevorzugt in einer Querschnittsdarstellung des Stomabeutel-Zubehörs von oben nach unten betrachtet das Klebeelement, das Substrat, das Sensorelement und der Absorber aufeinander folgen. Hierdurch können hervorstehende Abschnitte des Substrats oder des Absorbers, welches zu Unannehmlichkeiten des Benutzers führen können, beispielsweise indem sie an Gegenständen hängen bleiben, vermieden werden.

Das Substrat bildet bei der senkrechten Betrachtung einen Ringabschnitt aus und legt sich in dem Anwendungszustand zumindest teilweise um die Stomabeutelvorrichtung. Unter einem Ringabschnitt soll eine zusammenhängende Fläche verstanden werden, welche durch eine Innenlinie, eine Außenlinie und zwei Endlinien begrenzt ist. Die Innenlinie und die Außenlinie sind bevorzugt formidentisch, zueinander konzentrisch angeordnet und an ihren Endpunkten durch die Endlinien verbunden. Die Endlinien sind vorzugsweise gerade. Bevorzugt bildet der Ringabschnitt bei einer Erstreckung der Innenlinie und Außenlinie über 360° ihres Mittelpunkwinkels einen vollständigen Ring. Die Innenlinie und Außenlinie könnten Teile von Vielecken und der Ringabschnitt Teil eines Vieleckrings sein, bevorzugt sind die Innenlinie und Außenlinie Teile von Ovalen, bevorzugt Kreisen und der Ringabschnitt Teil eines Ovalrings, bevorzugt Kreisrings. Ein Innendurchmesser des Ringabschnitts ist bevorzugt von einer Größe der zu verwendenden Stomabeutelvorrichtung abhängig und bevorzugt dieser angepasst. Bevorzugt ist der Innendurchmesser des Ringabschnitts um ≤ 3 cm, weiter bevorzugt ≤ 2 cm, besonders bevorzugt ≤ 1 cm kleiner als ein Durchmesser der Hydrokolloidplatte. Bevorzugt ist der Innendurchmesser des Ringabschnitts ≥ 1 cm, weiterhin bevorzugt ≥ 1,5 cm, besonders bevorzugt ≥ 2 cm. Vorzugsweise ist der Innendurchmesser ≤ 10 cm. Bevorzugt ist ein Außendurchmesser des Ringabschnitts um ≥ 1 cm größer als der Innendurchmesser des Ringabschnitts. Darunter, dass sich das Substrat in dem Anwendungszustand zumindest teilweise um die Stomabeutelvorrichtung legt, soll verstanden werden, dass sich das Substrat in dem Anwendungszustand zumindest über einen Teil des Umfangs der Stomabeutelvorrichtung erstreckt und/oder dass sich in dem Anwendungszustand zumindest ein Teil des Substrats, vorzugsweise ein das Sensorelement aufweisender Teilbereich des Substrats, um die Stomabeutelvorrichtung legt. Besonders bevorzugt ist das Sensorelement in dem Anwendungszustand in einem Nahbereich eines äußeren Randes der Hydrokolloidplatte angeordnet. Bevorzugt weisen die Leiterbahnen des Sensorelements jeweils zumindest einen Leiterbahn-Ringabschnitt auf, dessen Durchmesser zwischen dem Innendurchmesser und Außendurchmesser des Ringabschnitts liegt. Hierdurch kann auf einfache Weise eine Leckage unabhängig davon, wo die Leckage auftritt, detektiert werden. Bevorzugt kann das Stomabeutel-Zubehör auf einfache Weise angebracht und wieder entfernt werden. Dies kann bevorzugt dadurch erreicht werden, dass der Klebstoff des Klebeelements eine Haftkraft in einem Bereich von 1 N/20 mm bis 30 N/20 mm gegen Stahl gemäß DIN EN 1464:2012-06 (90° Rollenschälprüfung) auf einer Zugprüfmaschine gemäß DIN EN ISO 527-1:2016-06 aufweist.

Bevorzugt erstreckt sich der Ringabschnitt bei der senkrechten Betrachtung über 360° seines Mittelpunktwinkels und bildet einen vollständigen Ring aus. In diesem Fall erstreckt sich das Sensorelement nur über einen Teil des Ringabschnitts und ein restlicher Teil des Ringabschnitts ist als eine dünne Brücke ausgebildet, welche dazu vorgesehen ist, den Ringabschnitt in Form zu halten. Bevorzugt ist die dünne Brücke des Ringabschnittes dehnbar ausgestaltet. Dies erleichtert ein Umlegen des Stomabeutel-Zubehörs um die Stomabeutelvorrichtung.

Besonders bevorzugt erstreckt sich der Ringabschnitt bei der senkrechten Betrachtung über höchstens 330°, vorteilhaft höchstens 300°, besonders vorteilhaft höchstens 270° und bevorzugt höchstens 240° seines Mittelpunktwinkels. Hierdurch kann ein leichtes Anbringen des Stomabeutel-Zubehörs erreicht werden, das sich aufgrund der Aussparung im Ringabschnitt das Stomabeutel-Zubehör auch um Gegenstände herum am der Stomabeutelvorrichtung anbringen lässt. Bevorzugt ist der Ringabschnitt als ein offener Ringabschnitt ausgebildet. Hierdurch kann ein "Einhaken" des Stomabeutel-Zubehörs, in welchem die Stomabeutelvorrichtung durch den offenen Teilabschnitt des Stomabeutel-Zubehörs geführt wird, ermöglicht werden, wodurch ein Verbinden des Stomabeutel-Zubehörs mit der Stomabeutelvorrichtung mit wenigen Handgriffen und in kürzester Zeit ermöglicht wird.

Das Stomabeutel-Zubehör weist bevorzugt eine Breite in einem Bereich von 5 bis 50 cm, weiter bevorzugt im Bereich von 6 bis 40 cm, besonders bevorzugt im Bereich von 7 bis 30 cm auf. Das Stomabeutel-Zubehör weist bevorzugt eine Länge in einem Bereich von 5 bis 100 cm, weiter bevorzugt im Bereich von 6 bis 70 cm, besonders bevorzugt im Bereich von 10 bis 50 cm auf. Das Stomabeutel-Zubehör kann jede Form aufweisen, die der Fachmann hierfür verwenden würde. Bevorzugt weist das Stomabeutel-Zubehör eine ovale oder kreisförmige Form auf.

Das Substrat weist bevorzugt eine Breite in einem Bereich von 1 bis 50 cm, weiter bevorzugt im Bereich von 2 bis 20 cm, besonders bevorzugt im Bereich von 5 bis 10 cm auf. Das Substrat weist bevorzugt eine Länge in einem Bereich von 2 bis 100 cm, weiter bevorzugt im Bereich von 5 bis 70 cm, besonders bevorzugt im Bereich von 10 bis 50 cm auf. Das Substrat kann jede Form aufweisen, die der Fachmann hierfür verwenden würde. Bevorzugt weist das Substrat eine ovale oder kreisförmige Form auf.

Der Absorber weist bevorzugt eine Breite in einem Bereich von 1 bis 50 cm, weiter bevorzugt im Bereich von 2 bis 20 cm, besonders bevorzugt im Bereich von 5 bis 10 cm auf. Der Absorber weist bevorzugt eine Länge in einem Bereich von 2 bis 100 cm, weiter bevorzugt im Bereich von 5 bis 70 cm, besonders bevorzugt im Bereich von 10 bis 50 cm auf. Der Absorber kann jede Form aufweisen, die der Fachmann hierfür verwenden würde. Bevorzugt weist der Absorber eine ovale oder kreisförmige Form auf.

Bevorzugt sind das Substrat und der Absorber zunächst lediglich aufeinander aufgelegt und in dem Anwendungszustand durch das Klebeelement fixiert. Besonders bevorzugt weist das Stomabeutel-Zubehör eine Fixiereinheit weist, welche das Substrat an dem Absorber fixiert und zumindest in einem Teilbereich zwischen dem Sensorelement und dem Absorber für Flüssigkeiten durchlässig ist. Bevorzugt erstreckt sich der flüssigkeitsdurchlässige Teilbereich ≥ 80 %, bevorzugt ≥ 90 % einer längsten Erstreckung des Sensorelements. Der Begriff "Fixiereinheit" umfasst in diesem Zusammenhang sowohl eine Gruppe von Fixierelementen zur Fixierung des Substrats an dem Absorber als auch Zwischenräume zwischen den Fixierelementen. Die Fixierelemente können zueinander identisch oder voneinander verschieden ausgebildet sein. Eine Anordnung der Fixierelemente zueinander kann unregelmäßig sein, bevorzugt sind die Fixierelemente zueinander regelmäßig angeordnet, beispielsweise sind die Fixierelemente bei der senkrechten Betrachtung neben dem Sensorelement entlanglaufend angeordnet. Bevorzugt weisen die Fixierelemente zumindest eine/n Schweißnaht, Schweißpunkt, Klammer und/oder Niete aufweisen, welche jeweils von dem Sensorelement beabstandet angeordnet sind. In diesem Fall ist der flüssigkeitsdurchlässige Teilbereich als Zwischenraum zwischen den Fixierelementen ausgebildet. Weiter bevorzugt ist die Fixiereinheit als ein zusammenhängender Klebefilm und/oder ein Klebeband ausgebildet, welches in sämtlichen Teilbereichen für Flüssigkeiten durchlässig ist. Hierdurch kann ein Verrutschen des Substrats relativ zu dem Absorber auch vor einem Verbinden des Stomabeutel-Zubehörs mit der Stomabeutelvorrichtung vermieden werden. Bevorzugt kann ein Einschränken der Funktion des Sensorelements durch die Fixiereinheit vermieden werden.

Besonders bevorzugt ist die Fixiereinheit als ein teilflächiger Klebefilm ausgebildet, wobei der flüssigkeitsdurchlässige Teilbereich als ein klebstofffreier Bereich der Fixiereinheit ausgebildet ist. Bevorzugt kann die Fixiereinheit aus einer Mehrzahl an Klebstoffpunkten und/oder Klebstoffstreifen bestehen. Weiter bevorzugt weisen die einzelnen Klebebereiche der Fixiereinheit beliebige andere Formen auf. Vorzugsweise weist die Fixiereinheit einen Klebstoff auf Haftkleberbasis, alternativ oder zusätzlich auf Strukturklebstoffbasis, auf. Hierdurch kann eine noch sicherere Konstruktion des Stomabeutel-Zubehörs erreicht werden und es kann ein Verrutschen des Substrats relativ zu dem Absorber auf besonders einfache und effiziente Weise vermieden werden. Besonders bevorzugt wird die Fixiereinheit mittels eines Liners auf das Substrat oder den Absorber aufgetragen, welcher im Anschluss abgezogen wird, um das Auflegen des jeweils anderen Fügepartners auf den teilflächigen Klebefilm zu ermöglichen.

Bevorzugt ist das Klebeelement als ein doppelseitiges Klebeband ausgebildet und das Stomabeutel-Zubehör weist eine zusätzliche Abdeckung auf, welche im Anwendungszustand das Klebeelement abdeckt. Besonders bevorzugt ist das Klebeband als eine selbstklebende Abdeckung ausgebildet, welche zumindest in dem Anwendungszustand auf einer dem Sensorelement abgewandten Seite des Substrats angeordnet ist. Bevorzugt weist das Klebeelement einen auf einer in dem Anwendungszustand dem Substrat zugewandten Seite einen Klebefilm und auf einer in dem Anwendungszustand dem Substrat abgewandten Seite einen Träger auf. Der Träger weist bevorzugt zumindest einen Zellstoff, Kunststoff und/oder Schaum auf. Vorzugsweise ist der Träger hydrophob oder weist eine hydrophobe Beschichtung auf. Hierdurch kann eine fälschliche Detektion einer Leckage durch ein Eindringen von Flüssigkeiten durch den Träger vermieden werden. Bevorzugt erstreckt sich das Klebeelement bei der senkrechten Betrachtung in dem Anwendungsbereich zumindest über das gesamte Stomabeutel-Zubehör und einen Randbereich der Stomabeutelvorrichtung. Es ist bevorzugt, dass das Klebeelement auch außerhalb des Anwendungszustands auf der dem Sensorelement abgewandten Seite des Substrats angeordnet ist. In diesem Fall weist das Stomabeutel-Zubehör vorzugsweise einen Release Liner auf, welcher einen Teil des Klebebereichs, welcher zu einem Auflegen auf die Haut des Patienten vorgesehen ist, vor dem Anbringen des Stomabeutel-Zubehörs abdeckt. Alternativ ist das Klebeelement erst während des Anbringens des Stomabeutel-Zubehörs auf der dem Sensorelement abgewandten Seite des Substrats angeordnet. Hierdurch kann das Verbinden des Stomabeutel-Zubehörs mit der Stomabeutelvorrichtung durch ein simples Auflegen und Glattstreichen des Stomabeutel-Zubehörs durchgeführt werden.

Vorzugsweise steht das Klebeelement bei der senkrechten Betrachtung in dem Anwendungszustand zu beiden Seiten des Substrats um zumindest 30%, bezogen auf eine Breite des Substrats, über das Substrat hervor. Es wäre möglich, dass das Klebeelement entlang des Verlaufs des Substrats unterschiedlich weit hervorsteht. Vorzugsweise ist das Klebeelement in der senkrechten Betrachtung als ein Ringabschnitt und besonders bevorzugt formidentisch zu dem Substrat ausgebildet. Hierdurch kann eine robuste Verbindung des Stomabeutel-Zubehörs mit der Stomabeutelvorrichtung erreicht werden. Vorteilhaft kann das Klebeelement gleichzeitig an der Hydrokolloidplatte der Stomabeutelvorrichtung, dem Substrat des Stomabeutel-Zubehörs und der Haut des Benutzers haften.

Bevorzugt weist das Stomabeutel-Zubehör genau ein Sensorelement auf. Besonders bevorzugt weist das Stomabeutel-Zubehör eine Mehrzahl von Sensorelementen auf, welche zueinander konzentrisch angeordnet sind. Bevorzugt weist das Stomabeutel-Zubehör zwei oder drei Sensorelemente auf. Besonders bevorzugt teilen sich benachbarte Sensorelemente eine der Leiterbahnen. Vorzugsweise sind die Sensorelemente in regelmäßigen radialen Abständen voneinander auf dem Substrat angeordnet. Hierdurch kann zusätzlich zu der Information, dass eine Leckage auftritt, eine Aussage über die Schwere der Leckage gemacht werden, indem festgestellt wird, bei welchem der Sensorelemente wieviel Feuchtigkeit ankommt. Vorteilhaft kann eine Redundanz geschaffen werden, die eine Detektion einer Leckage erlaubt, auch wenn eines der Sensorelemente defekt sein sollte.

Ein weiterer Gegenstand der Erfindung betrifft ein System mit zumindest einer Stomabeutelvorrichtung und mit zumindest einem Stomabeutel-Zubehör vorgeschlagen. Hierdurch kann ein System bereitgestellt werden, welches verbesserte Eigenschaften hinsichtlich einer Konstruktion aufweist.

Ein weiterer Gegenstand der Erfindung betrifft ein Verfahren zur Anwendung eines Stomabeutel-Zubehörs mit einer Stomabeutelvorrichtung, bevorzugt eines zuvor beschriebenen, erfindungsgemäßen Stomabeutel-Zubehörs. Hierbei wird das Stomabeutel-Zubehör um einen Rand einer Hydrokolloidplatte der Stomabeutelvorrichtung positioniert und durch ein Auflegen eines Klebeelements des Stomabeutel-Zubehörs auf der Hydrokolloidplatte und auf einer die Stomabeutelvorrichtung umgebenden Haut befestigt wird. Hierdurch kann ein Anbringen und Entfernen des Stomabeutel-Zubehörs auf eine einfache und intuitive Weise ermöglicht werden.

Sämtliche Angaben für das zuvor beschriebene, erfindungsgemäße Stomabeutel-Zubehör und die Stomabeutelvorrichtung, wie beispielsweise dort genannten Maße, Eigenschaften, Zusammensetzungen und Formen gelten auch für das Verfahren zur Anwendung des Stomabeutel-Zubehörs gleichermaßen.

In einer bevorzugten Ausführungsform des Verfahrens, liegen ein Absorber und ein Substrat des Stomabeutel-Zubehörs in einem unbefestigten Zustand des Stomabeutel-Zubehörs voneinander gelöst vor und das Substrat wird erst während des Auflegens des Klebeelements mit dem Klebeelement fixiert. Hierdurch können einzelne Bestandteile des Stomabeutel-Zubehörs beispielsweise im Fall eines Defekts leicht ausgetauscht werden. Um ein fehlerhaftes Anbringen des Stomabeutel-Zubehörs zu vermeiden, wird vorgeschlagen, dass der Absorber und das Substrat in dem unbefestigten Zustand miteinander verbunden sind und das Substrat vor dem Auflegen des Klebeelement auf der Hydrokolloidplatte mit dem Klebeelement verbunden wird. Hierdurch kann das Stomabeutel-Zubehör direkt nach dem Kauf von dem Benutzer mit wenigen Handgriffen angebracht werden.

Das Stomabeutel-Zubehör und das Verfahren sollen hierbei nicht auf die oben beschriebene Anwendung und Ausführungsform beschränkt sein. Bevorzugt können das Stomabeutel-Zubehör und das Verfahren zu einer Erfüllung einer hierin beschriebenen Funktionsweise eine von einer hierin genannten Anzahl von einzelnen Elementen, Bauteilen, Einheiten oder Verfahrensschritten abweichende Anzahl aufweisen.

Ein Verfahren zur Herstellung des Stomabeutel-Zubehörs weist vorzugsweise mindestens die folgenden Schritte auf: 1) Bereitstellen des Substrats, 2) Bedrucken des Substrats mit dem Sensorelement, 3) Auflegen und Fixieren des Substrats auf dem Absorber. Bevorzugt kann das Verfahren zur Herstellung des Stomabeutel-Zubehörs einen zusätzlichen Schritt aufweisen, in welchem das Substrat und der Absorber durch ein Auflegen des Klebeelements auf das Substrat fixiert werden. Hierdurch kann ein Stomabeutel-Zubehör hergestellt werden, welcher einen erhöhten Komfort und eine verbesserte Flexibilität aufweist.

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine schematische Darstellung eines Systems mit einer Stomabeutelvorrichtung und einem erfindungsgemäßen Stomabeutel-Zubehör in einer Querschnittsansicht,
- Fig. 2: eine schematische Darstellung des Systems in einer Draufsicht,
- Fig. 3: eine schematische Darstellung eines Teils des Systems in einer näheren Draufsicht und
- Fig. 4: ein schematisches Verlaufsdiagramm eines Verfahrens zur Anwendung des Stomabeutel-Zubehörs.

Aus Gründen der Übersichtlichkeit wird mehreren zueinander identischen Objekten in den Figuren jeweils nur ein Bezugszeichen zugeordnet.

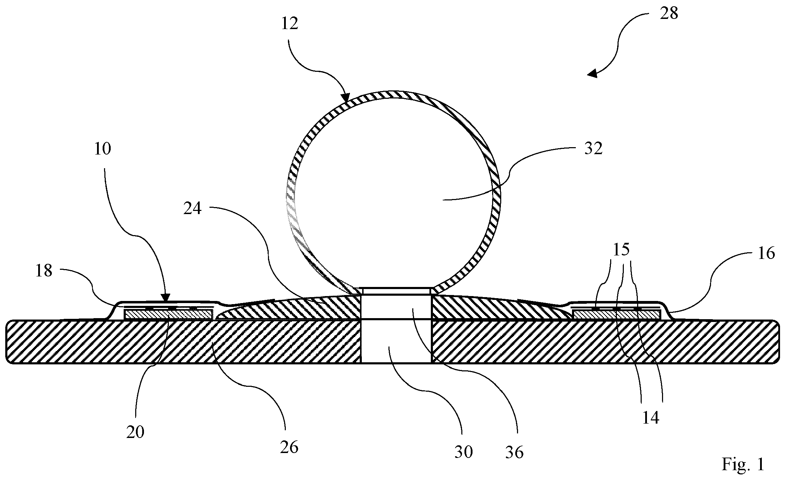

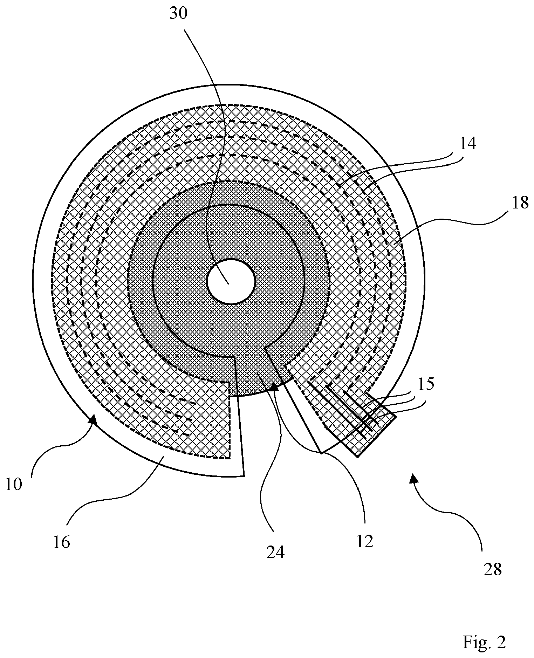

Die Figuren 1 und 2 zeigen ein System 28. Das System 28 befindet sich in einem Anwendungszustand. Das System 28 weist eine Stomabeutelvorrichtung 12 auf. Die Stomabeutelvorrichtung 12 ist über eine Öffnung 36 an einem Stoma 30 eines Benutzers (nicht dargestellt) angeordnet. Die Stomabeutelvorrichtung 12 weist eine Hydrokolloidplatte 24 auf. Die Hydrokolloidplatte 24 ist auf eine Haut 26 des Benutzers aufgeklebt und umschließt das Stoma 30. Die Stomabeutelvorrichtung 12 weist einen Stomabeutel 32 auf (welcher aus Gründen der Übersichtlichkeit nur in Figur 1 dargestellt ist). Der Stomabeutel 32 ist zur Aufnahme von Stoffen, welche aus dem Stoma 30 entweichen, vorgesehen und mit der Hydrokolloidplatte 24 verbunden.

Das System 28 weist ein Stomabeutel-Zubehör 10 auf. Das Stomabeutel-Zubehör 10 weist ein Substrat 18 auf. Das Substrat 18 ist als eine flexible Folie ausgebildet. Das Substrat 18 weist eine Bruchdehnung von 500 % auf. Das Substrat 18 weist eine Bruchspannung von 50 MPa auf. Das Substrat 18 besteht aus Polycarbonat. Das Stomabeutel-Zubehör 10 weist ein Sensorelement 14 auf. Das Sensorelement 14 ist als ein aufgedruckter Sensor ausgebildet. Das Sensorelement 14 besteht aus einer anorganischen Tinte, welche Silberpartikel aufweist. Das Sensorelement 14 dient einer Erkennung einer Leckage der Stomabeutelvorrichtung 12. Das Sensorelement 14 ist als ein Feuchtigkeitssensor ausgebildet. Das Sensorelement 14 ist auf das Substrat 18 aufgedruckt. Das Sensorelement 14 weist zwei Leiterbahnen auf. Die Leiterbahnen sind als offene Leiterbahnen ausgebildet, welche umkehrfrei und frei von Vorsprüngen sind. Alternativ könnten die Leiterbahnen geschlossen sein, Umkehrpunkte aufweisen und/oder Vorsprünge aufweisen.

Das Stomabeutel-Zubehör 10 weist drei Leiterbahnen 15 auf. Die Leiterbahnen 15 sind zueinander identisch ausgebildet. Alternativ könnten die Leiterbahnen 15 auch voneinander verschieden ausgebildet sein. Das Stomabeutel-Zubehör 10 weist ein weiteres Sensorelement auf. Das weitere Sensorelement teilt sich eine der Leiterbahnen 15 mit dem Sensorelement 14. Das Sensorelement 14 und das weitere Sensorelement sind zueinander konzentrisch angeordnet. Das Sensorelement 14 und das weitere Sensorelement weisen einen gleichmäßigen radialen Abstand zueinander auf.

Das Stomabeutel-Zubehör 10 weist einen Absorber 20 auf. Der Absorber 20 besteht aus einem Vliesstoff. Alternativ oder zusätzlich kann der Absorber 20 auch einen Schaum und/oder einen Zellstoff aufweisen. Der Absorber 20 weist eine maximal aufnehmbare Flüssigkeitsmenge von 50 ml auf. Der Absorber 20 weist eine maximale Porengröße von 100 µm auf. Der Absorber 20 ist auf die Haut 26 des Benutzers aufgelegt. Das Substrat 18 ist auf den Absorber 20 aufgelegt. Das Sensorelement 14 ist zwischen dem Substrat 18 und dem Absorber 20 angeordnet. Das Substrat 18 und der Absorber 20 sind zueinander deckungsgleich ausgebildet. Das Substrat 18, das Sensorelement 14 und der Absorber 20 sind relativ zueinander in einer Sandwich-Bauweise angeordnet.

Das Stomabeutel-Zubehör 10 weist ein Klebeelement 16 auf. Das Klebeelement 16 dient einer lösbaren Verbindung des Stomabeutel-Zubehörs 10 mit der Stomabeutelvorrichtung 12. Das Klebeelement 16 ist als eine selbstklebende Abdeckung ausgebildet. Das Klebeelement 16 weist eine Abdeckungsseite und eine Klebeseite auf. Das Klebeelement 16 weist einen mit einem Polyurethanklebstoff, ausgestatteten Klebebereich auf, welcher sich über die gesamte Klebeseite erstreckt. Das Klebeelement 16 ist auf einer dem Sensorelement 14 abgewandten Seite des Substrats 18 angeordnet. Das Klebeelement 16 ist auf das Substrat 18 aufgelegt (in Figur 1 wurde aus Gründen der Übersichtlichkeit ein Spalt zwischen dem Klebeelement 16 und dem Substrat 18 gelassen). Das Klebeelement 16 steht zu beiden Seiten des Substrats 18 um etwa 30 % einer Breite des Substrats 18 hervor. Das Klebeelement 16 ist auf einen Rand der Hydrokolloidplatte 24 aufgelegt. Das Klebeelement 16 ist auf die Haut 26 des Benutzers aufgelegt.

Das Substrat 18 bildet einen Ringabschnitt aus (siehe Figur 2). Das Substrat 18 legt sich teilweise um die Stomabeutelvorrichtung 12. Der Ringabschnitt erstreckt sich bei der senkrechten Betrachtung über etwa 320°.

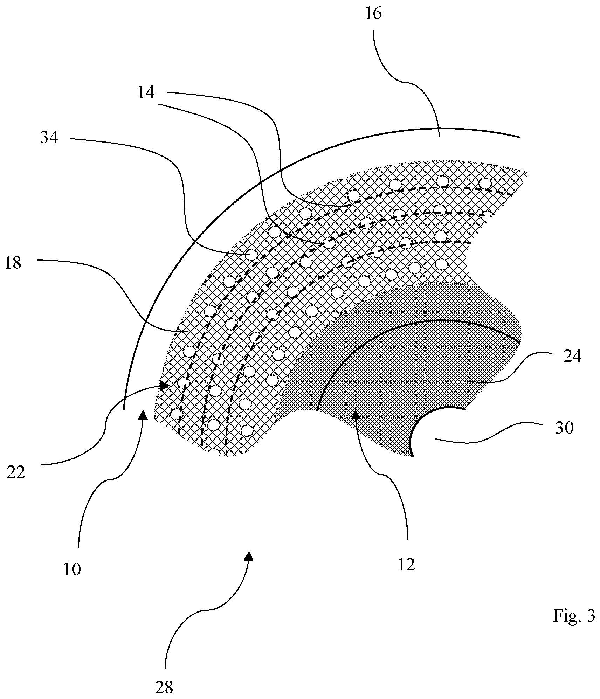

Das Stomabeutel-Zubehör 10 weist eine Fixiereinheit 22 auf, welche in Figur 3 dargestellt ist. Die Fixiereinheit 22 fixiert das Substrat 18 an dem Absorber 20. Die Fixiereinheit 22 ist in einem Teilbereich zwischen dem Sensorelement 14 und dem Absorber 20 für Flüssigkeiten durchlässig. Die Fixiereinheit 22 ist als ein teilflächiger Klebefilm ausgebildet. Die Fixiereinheit 22 umfasst eine Gruppe von Fixierelementen 34 sowie Zwischenräume zwischen den Fixierelementen 34. Die Fixierelemente 34 sind als Klebepunkte ausgebildet, alternativ könnten die Fixierelemente 34 auch als Klebestreifen ausgebildet sein. Der Teilbereich ist als ein klebstofffreier Bereich der Fixiereinheit 22 ausgebildet.

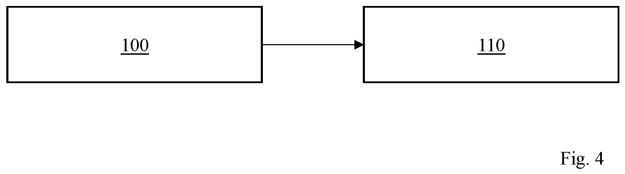

Figur 4 zeigt ein schematisches Verlaufsdiagramm eines Verfahrens zur Anwendung des Stomabeutel-Zubehörs 10 mit der Stomabeutelvorrichtung 12. In einem Vorbereitungsschritt 100 wird das Substrat 18 mit dem Klebeelement 16 fixiert, indem es gegen den Klebebereich des Klebeelements 16 gedrückt wird. Der Vorbereitungsschritt 100 kann entweder von dem Benutzer selbst oder während der Herstellung des Stomabeutel-Zubehörs durchgeführt werden.

In einem Auflageschritt 110 wird das Stomabeutel-Zubehör 10 um den Rand der Hydrokolloidplatte 24 positioniert und durch ein Auflegen des Klebeelements 16 auf der Hydrokolloidplatte 24 und der Haut 26 befestigt. Hierbei folgt der Auflageschritt 110 dem Vorbereitungsschritt 100. Alternativ könnte dem Auflageschritt 110 kein anderer Schritt folgen, in diesem Fall würde der Auflageschritt 110 zusätzlich ein Auflegen des Substrats 18 und des Absorbers 20 auf die Haut 26 vor dem Auflegen des Klebeelements 22 umfassen.

### Bezugszeichen

- 10: Stomabeutel-Zubehör
- 12: Stomabeutelvorrichtung
- 14: Sensorelement
- 15: Leiterbahn
- 16: Klebeelement
- 18: Substrat
- 20: Absorber
- 22: Fixiereinheit
- 24: Hydrokolloidplatte
- 26: Haut
- 28: System
- 30: Stoma
- 32: Stomabeutel
- 34: Fixierelement
- 36: Öffnung
- 100: Vorbereitungsschritt
- 110: Auflageschritt

## Patentansprüche

1. Stomabeutel-Zubehör (10) zur Verwendung mit zumindest einer Stomabeutelvorrichtung (12), mit zumindest einem Sensorelement (14) zur Erkennung einer Leckage der Stomabeutelvorrichtung (12) in zumindest einem Anwendungszustand, mit zumindest einem Substrat (18), welches das Sensorelement (14) trägt, und mit zumindest einen Absorber (20), wobei das Sensorelement (14) bei einer Betrachtung senkrecht auf eine Haupterstreckungsebene des Substrats (18) zwischen dem Substrat (18) und dem Absorber (20) angeordnet ist, **gekennzeichnet durch** zumindest ein Klebeelement (16) zu einem lösbaren Kleben des Stomabeutel-Zubehörs (10) an eine Hydrokolloidplatte (24) der Stomabeutelvorrichtung (14) in dem Anwendungszustand, wobei das Substrat (18) bei der senkrechten Betrachtung einen Ringabschnitt ausbildet und sich in dem Anwendungszustand zumindest teilweise um die Stomabeutelvorrichtung (12) legt.

2. Stomabeutel-Zubehör (10) nach Anspruch 1, **dadurch gekennzeichnet, dass** das Sensorelement (14) als ein aufgedruckter Sensor ausgebildet ist und das Substrat (18) zumindest eine, bevorzugt zwei, besonders bevorzugt alle der folgenden Eigenschaften aufweist:
1) eine Bruchdehnung ≥ 300 % und ≤ 800 %
2) eine Bruchspannung ≥ 30 MPa und ≤ 100 MPa
3) Beinhalten eines Polymers ausgewählt aus der Gruppe bestehend aus einem Polyester, einem Polycarbonat, einem Co-Polycarbonat, einem Polyurethan, einem thermoplastischen Polyurethan, einem Acrylnitril-Butadien-Styrol-Copolymer, einem Polyethylenterephthalat, einem Polyamin, einem Polyamid, einem Polystyrol und einem Polysiloxan oder Blends oder Mischung aus mindestens zwei hiervon.

3. Stomabeutel-Zubehör (10) nach Anspruch 1 oder 2, **dadurch gekennzeichnet, dass** der Absorber (20) zumindest eine, bevorzugt alle der folgenden Eigenschaften aufweist:
1) eine maximal aufnehmbare Flüssigkeitsmenge von ≥ 40 ml
2) eine Porengröße von ≤ 120 µm.

4. Stomabeutel-Zubehör (10) nach einem der vorhergehenden Ansprüche, **dadurch gekennzeichnet, dass** das Substrat (18) und der Absorber (20) bei einer senkrechten Betrachtung zumindest im Wesentlichen zueinander deckungsgleich ausgebildet sind.

5. Stomabeutel-Zubehör (10) nach einem der vorhergehenden Ansprüche, **dadurch gekennzeichnet, dass** sich der Ringabschnitt bei der senkrechten Betrachtung über höchstens 330° seines Mittelpunktwinkels erstreckt.

6. Stomabeutel-Zubehör (10) nach einem der vorhergehenden Ansprüche, **gekennzeichnet durch** eine Fixiereinheit (22), welche das Substrat (18) an dem Absorber (20) fixiert und zumindest in einem Teilbereich zwischen dem Sensorelement (14) und dem Absorber (20) für Flüssigkeiten durchlässig ist.

7. Stomabeutel-Zubehör (10) nach Anspruch 6, **dadurch gekennzeichnet, dass** die Fixiereinheit (22) als ein teilflächiger Klebefilm ausgebildet ist, wobei der Teilbereich als ein klebstofffreier Bereich der Fixiereinheit (22) ausgebildet ist.

8. Stomabeutel-Zubehör (10) nach einem der vorhergehenden Ansprüche, **dadurch gekennzeichnet, dass** das Klebeelement (16) als eine selbstklebende Abdeckung ausgebildet ist, welche zumindest in dem Anwendungszustand auf einer dem Sensorelement (14) abgewandten Seite des Substrats (18) angeordnet ist.

9. Stomabeutel-Zubehör (10) nach einem der vorhergehenden Ansprüche, **dadurch gekennzeichnet, dass** das Klebeelement (16) bei der senkrechten Betrachtung in dem Anwendungszustand zu beiden Seiten des Substrats (18) um zumindest 30%, bezogen auf eine Breite des Substrats (18), über das Substrat (18) hervorsteht.

10. Stomabeutel-Zubehör (10) nach einem der vorhergehenden Ansprüche, **gekennzeichnet durch** eine Mehrzahl von Sensorelementen (14), welche zueinander konzentrisch angeordnet sind.

11. System mit zumindest einer Stomabeutelvorrichtung (12) und mit zumindest einem Stomabeutel-Zubehör (10) nach einem der vorhergehenden Ansprüche.

12. Verfahren zur Anwendung eines Stomabeutel-Zubehörs (10) nach einem der Ansprüche 1 bis 10 mit einer Stomabeutelvorrichtung (12), **dadurch gekennzeichnet, dass** das Stomabeutel-Zubehör (10) um einen Rand einer Hydrokolloidplatte (24) der Stomabeutelvorrichtung (12) positioniert und durch ein Auflegen eines Klebeelements (16) des Stomabeutel-Zubehörs (10) auf der Hydrokolloidplatte (24) und auf einer die Stomabeutelvorrichtung (12) umgebenden Haut (26) befestigt wird.

13. Verfahren nach Anspruch 12, **dadurch gekennzeichnet, dass** ein Absorber (20) und ein Substrat (18) des Stomabeutel-Zubehörs (10) in einem unbefestigten Zustand des Stomabeutel-Zubehörs (10) miteinander verbunden sind und das Substrat (18) vor dem Auflegen des Klebeelements (16) auf der Hydrokolloidplatte (24) mit dem Klebeelement (16) verbunden wird.

## Claims

1. Ostomy pouch accessory (10) for usage with at least one ostomy pouch device (12), comprising at least one sensor element (14) for detection of a leakage of the ostomy pouch device (12) in at least one applied state, further comprising at least one substrate (18) carrying the sensor element (14), and further comprising at least one absorber (20), wherein the sensor element (14), in a perpendicular view onto a main extension plane of the substrate (18), is arranged between the substrate (18) and the absorber (20), **characterized by** at least one adhesive element (16) configured for a detachable adhering of the ostomy pouch accessory (10) to a hydrocolloid plate (24) of the ostomy pouch device (14) in the applied state, wherein the substrate (18) forms a ring section in the perpendicular view and surrounds the ostomy pouch device (12) at least partially in the applied state.

2. Ostomy pouch accessory (10) according to claim 1, **characterized in that** the sensor element (14) is a printed sensor and the substrate (18) exhibits at least one, preferably two, especially preferably all of the following properties:
1) an elongation at break ≥ 300 % and ≤ 800 %
2) a breaking stress ≥ 30 MPa and ≤ 100 MPa
3) comprising a polymer selected from the group consisting of a polyester, a polycarbonate, a co-polycarbonate, a polyurethane, a thermoplastic polyurethane, an acrylonitrile-butadiene-styrene-copolymer, a polyethylene terephthalate, a polyamine, a polyamide, a polystyrene and a polysiloxane or blend or mixture of at least two thereof.

3. Ostomy pouch accessory (10) according to claim 1 or 2, **characterized in that** the absorber (20) exhibits at least one, preferably all of the following properties:
1) a maximum liquid absorption of ≥ 40 ml
2) a pore size of ≤ 120 µm.

4. Ostomy pouch accessory (10) according to any of the preceding claims, **characterized in that** the substrate (18) and the absorber (20) are at least substantially congruent in the perpendicular view.

5. Ostomy pouch accessory (10) according to any of the preceding claims, **characterized in that** the ring section extends over at most 330° of its central angle in the perpendicular view.

6. Ostomy pouch accessory (10) according to any of the preceding claims, **characterized by** a fixation unit (22) attaching the substrate (18) to the absorber (20), the fixation unit (22) being permeable for fluids in at least a partial area between the sensor element (14) and the absorber (20).

7. Ostomy pouch accessory (10) according to claim 6, **characterized in that** the fixation unit (22) is a partially adhesive film, the partial area being a section of the fixation unit (22) which is free of adhesive.

8. Ostomy pouch accessory (10) according to any of the preceding claims, **characterized in that** the adhesive element (16) is a self-adhesive cover which, at least in the applied state, is arranged on a side of the substrate (18) opposite the sensor element (14).

9. Ostomy pouch accessory (10) according to any of the preceding claims, **characterized in that** the adhesive element (16) protrudes past the substrate (18) on both sides of the substrate (18) by at least 30 % of a width of the substrate (18) in the perpendicular view.

10. Ostomy pouch accessory (10) according to any of the preceding claims, **characterized by** a multitude of sensor elements (14) arranged concentrically.

11. System with at least one ostomy pouch device (12) and at least one ostomy pouch accessory (10) according to any of the preceding claims.

12. Method of using an ostomy pouch accessory (10) according to one of the claims 1 to 10 with an ostomy pouch device (12), **characterized in that** the ostomy pouch accessory (10) is arranged around a rim of a hydrocolloid plate (24) of the ostomy pouch device (12) and attached by placing an adhesive element (16) of the ostomy pouch accessory (10) onto the hydrocolloid plate (24) and a skin (26) surrounding the ostomy pouch device (12).

13. Method according to claim 12, **characterized in that** an absorber (20) and a substrate (18) of the ostomy pouch accessory (10) are attached to each other in an un-applied state of the ostomy pouch accessory (10) and **in that** the substrate (18) and the adhesive element (16) are attached to each other before the adhesive element (16) is placed onto the hydrocolloid plate (24).

## Revendications

1. Accessoire de poche de stomie (10) destiné à être utilisé avec au moins un dispositif de poche de stomie (12), comprenant au moins un capteur (14) pour détecter une fuite du dispositif de poche de stomie (12) dans au moins un état appliqué, comprenant en outre au moins un substrat (18) portant le capteur (14), et comprenant en outre au moins un absorbeur (20), l'élément capteur (14), vu perpendiculairement à un plan d'extension principal du substrat (18), étant disposé entre le substrat (18) et l'absorbeur (20), **caractérisé par** au moins un élément adhésif (16) configuré pour une adhésion détachable de l'accessoire de poche de stomie (10) à une plaque hydrocolloïde (24) du dispositif de poche de stomie (14) dans l'état appliqué, le substrat (18) formant une section annulaire dans la vue perpendiculaire et entourant au moins partiellement le dispositif de poche de stomie (12) dans l'état appliqué.

2. Accessoire de poche de stomie (10) selon la revendication 1, **caractérisé en ce que** l'élément capteur (14) est un capteur imprimé et que le substrat (18) présente au moins une, de préférence deux, et de préférence toutes les propriétés suivantes :
1) un allongement à la rupture ≥ 300 % et ≤ 800 %
2) une contrainte à la rupture ≥ 30 MPa et ≤ 100 MPa
3) comprenant un polymère choisi dans le groupe constitué par un polyester, un polycarbonate, un co-polycarbonate, un polyuréthane, un polyuréthane thermoplastique, un copolymère acrylonitrile-butadiène-styrène, un polyéthylène téréphtalate, une polyamine, un polyamide, un polystyrène et un polysiloxane ou un mélange ou une combinaison d'au moins deux d'entre eux.

3. Accessoire de poche de stomie (10) selon la revendication 1 ou 2, **caractérisé en ce que** l'absorbeur (20) présente au moins une, de préférence toutes les propriétés suivantes :
1) Absorption maximale de liquide ≥ 40 ml
2) Taille des pores ≤ 120 µm

4. Accessoire de poche de stomie (10) selon l'une quelconque des revendications précédentes, **caractérisé en ce que** le substrat (18) et l'absorbeur (20) sont au moins sensiblement congruents dans la vue perpendiculaire.

5. Accessoire de poche de stomie (10) selon l'une quelconque des revendications précédentes, **caractérisé en ce que** la section annulaire s'étend sur au plus 330° de son angle central en vue perpendiculaire.

6. Accessoire de poche de stomie (10) selon l'une quelconque des revendications précédentes, **caractérisé par** une unité de fixation (22) fixant le substrat (18) à l'absorbeur (20), l'unité de fixation (22) étant perméable aux fluides dans au moins une zone partielle entre l'élément capteur (14) et l'absorbeur (20).

7. Accessoire de poche de stomie (10) selon la revendication 6, **caractérisé en ce que** l'unité de fixation (22) est un film partiellement adhésif, la zone partielle étant une section de l'unité de fixation (22) exempte d'adhésif.

8. Accessoire de poche de stomie (10) selon l'une quelconque des revendications précédentes, **caractérisé en ce que** l'élément adhésif (16) est un film autocollant qui, au moins à l'état appliqué, est disposé sur un côté du substrat (18) opposé à l'élément capteur (14).

9. Accessoire de poche de stomie (10) selon l'une quelconque des revendications précédentes, **caractérisé en ce que** l'élément adhésif (16) dépasse du substrat (18) des deux côtés de celui-ci d'au moins 30 % de sa largeur en vue perpendiculaire.

10. Accessoire de poche de stomie (10) selon l'une quelconque des revendications précédentes, **caractérisé par** une multitude d'éléments capteurs (14) disposés concentriquement.

11. Système comprenant au moins un dispositif de poche de stomie (12) et au moins un accessoire de poche de stomie (10) selon l'une quelconque des revendications précédentes.

12. Procédé d'utilisation d'un accessoire de poche de stomie (10) selon l'une des revendications 1 à 10 avec un dispositif de poche de stomie (12), **caractérisé en ce que** l'accessoire de poche de stomie (10) est disposé autour du bord d'une plaque hydrocolloïde (24) du dispositif de poche de stomie (12) et fixé par la pose d'un élément adhésif (16) de l'accessoire de poche de stomie (10) sur la plaque hydrocolloïde (24) et sur une peau (26) entourant le dispositif de poche de stomie (12).

13. Procédé selon la revendication 12, **caractérisé en ce qu'**un absorbeur (20) et un substrat (18) de l'accessoire de poche de stomie (10) sont fixés l'un à l'autre lorsque l'accessoire de poche de stomie (10) est non appliqué, et **en ce que** le substrat (18) et l'élément adhésif (16) sont fixés l'un à l'autre avant la pose de l'élément adhésif (16) sur la plaque hydrocolloïde (24).
